# EUROPÄISCHE PATENTSCHRIFT

(11) **EP 1 385 435 B1**
(45) Veröffentlichungstag und Bekanntmachung des Hinweises auf die Patenterteilung: **13.12.2006**
(21) Anmeldenummer: 02742980.2
(22) Anmeldetag: 10.05.2002
(51) Int. Cl.: A61B 17/66

(54) **VORRICHTUNG FÜR KIEFERORTHOPÄDISCHE UND/ODER KIEFERCHIRURGISCHE ZWECKE**
ORTHODONTIC AND/OR OROSURGICAL DEVICE
DISPOSITIF D'ORTHOPEDIE ET/OU DE CHIRURGIE DE LA MACHOIRE

(30) Priorität: 10.05.2001 CH 8452001
(43) Veröffentlichungstag der Anmeldung: 04.02.2004
(73) Patentinhaber: Minoretti, Roger, 8006 Zürich (CH); Merz, Beat R., 8049 Zürich (CH)
(72) Erfinder: MINORETTI, Roger, 8006 Zürich (CH); MERZ, Beat, R., 8049 Zürich (CH); TRIACA, Albino, 8006 Zürich (CH)
(74) Vertreter: Grimm, Ekkehard
(86) Internationale Anmeldenummer: PCT/EP2002/005146
(87) Internationale Veröffentlichungsnummer: WO 2002/089682

(56) Entgegenhaltungen:
- EP-A- 0 786 975
- EP-A- 0 947 177
- DE-A- 19 859 503
- US-A- 5 052 930
- US-A- 5 399 088
- US-A- 5 697 779
- US-A- 5 882 193
- US-A- 5 885 283
- US-B1- 6 302 687

## Beschreibung

Die vorliegende Erfindung betrifft eine Vorrichtung für kieferorthopädische und/oder kieferchirurgische Zwecke gemäß dem Oberbegriff des Anspruchs 1.

Eine solche Vorrichtung ist aus der US-A-5 885 283 bekannt.

Solche Vorrichtungen kommen zum Einsatz im Bereich der Kieferorthopädie und der Kieferchirurgie.

### Stand der Technik

In der kieferorthopädisch/orthodontischen Behandlung von Fehlstellungen der Zähne werden diese mittels relativ geringer Kräfte in der Größenordnung von ein paar wenigen Newton im Alveolarkamm bewegt und/oder verkippt. Diese Behandlung kann teilweise jahrelang dauern, weil sich die Zähne nur langsam bewegen lassen.

Normalerweise werden die Kräfte durch zwischen den Zähnen aufgespannte Federn erzeugt, z.B. um die benachbarten Zähne in eine Lücke zu ziehen. Dies nennt man auch eine parodontale Verankerung, weil die zur Verankerung herbeigezogenen Zähne sich ebenfalls bewegen, und es ohne zusätzliche Verankerungshilfe, z.B. in der Form einer extraoralen Apparatur, nicht möglich ist, nur die Zähne an dem einen Ende der Federn zu bewegen und die anderen ortsfest zu halten.

In vielen Fällen ist es aber nicht erwünscht, dass sich die Verankerungszähne bewegen, z.B. weil man eine Lücke nur mit den distal davon stehenden Zähnen schließen will. In anderen Fällen, z.B. bei Nichtanlagen von Zähnen oder bei parodontaler Schädigung, kann es nahezu unmöglich sein, zur Verankerung geeignete Zähne zu finden. Dann verbleiben nur noch Maßnahmen über extraorale Verankerungshilfen, sogenannte Headgears, oder die Verankerung über eine an den Kieferknochen oder benachbarten Knochen festgeschraubte Apparatur.

Die vorgenannten Headgears sind zum einen nicht für alle Behandlungen geeignet, zum anderen ist die Patientenmitarbeit bei diesen unästhetischen, störenden Vorrichtungen am Kopf meist sehr schlecht, so dass das Behandlungsergebnis gefährdet wird.

Knochenabgestützte Verankerungen haben den Vorteil, dass sie sich unter den geringen orthodontischen Kräften nicht bewegen und deshalb als absolute Ankerpunkte dienen können.

Solche knochenabgestützte Verankerungen sind aus verschiedenen Dokumenten bekannt; so beschreibt z.B. die *EP-A-0,786,975* ein spezielles Implantat, das in der Gaumennaht fixiert wird und dort für die Knochenabstützung von Verankerungszähnen im Oberkiefer dient.

Darüber hinaus beschreibt die *US 5,921,774* andere, spezielle Verankerungsschrauben oder Stifte, die zwischen den Zahnwurzeln gesetzt werden und als Verankerung für Zähne oder für kieferorthopädische Apparaturen dienen können.

Allen diesen Verankerungen ist der Nachteil gemeinsam, dass sie nur eine punktförmige Abstützung bieten, die aus Gründen der Anatomie meist nicht genau dort zu liegen kommt, wo eigentlich die Stabilisierung benötigt wird oder der Federkraftvektor hinzeigen sollte. Deshalb müssen solche Verankerungen oft mit aufwendigen Apparaturen kombiniert werden, um dann die gewünschte Verankerung oder Kraftwirkung entfalten zu können (siehe die Veröffentlichung Männchen R. "A new supraconstruction for palatal orthodontic implants", J Clin Orthod 1999; 33: 373-382).

Während sich im Oberkiefer noch der harte Gaumen bzw. dessen zentrale Sutur anbietet, kann im Unterkiefer bei den bestehenden Verankerungen in der Regel nur der Alveolarkamm herangezogen werden. Wegen der vorhandnen Bezahnung müssen die schraubenoder pinförmigen Verankerungen zwischen den Zahnwurzeln angebracht werden, was zum einen schwierig und für die Wurzeln gefährlich sein kann und zum anderen oft Probleme mit den Weichteilen mit sich bringt.

Die *EP-A-0 947 177* zeigt ein Zahnreguliersystem, mit dem die Stellung der Zähne korrigiert wird. Als Besonderheit dieses Systems ist ein Knochenanker in Form einer Knochenschraube vorgesehen, an der die Zahnregulierteile gehalten sind; gegen diesen Knochenanker unmittelbar oberhalb der Zähne (zwischen den Zahnwurzeln) kann ein Seil- oder Drahtsystem verspannt werden. Obwohl das System in den Figuren nur im Bereich der Zähne des Oberkiefers dargestellt ist, erscheint es möglich, dass dieses System zur Zahnregulierung auch im Bereich des Unterkiefers verwendbar ist.

Der vorliegenden Erfindung liegt daher die Aufgabe zugrunde, eine Vorrichtung für kieferorthopädische und/oder kieferchirurgische Zwecke zu schaffen, die die vorstehenden Nachteile vermeidet und insbesondere eine optimal einrichtbare Abstützung für den jeweiligen kieferorthopädischen oder kieferchirurgischen Eingriff bietet.

In der kieferchirurgischen Behandlung mit Distraktion von Knochensegmenten werden solche Knochensegmente vom restlichen Knochen abgetrennt und anschliessend der entstehende Heilungs-Callus distrahiert, um neue Knochenmasse zu gewinnen.

Ein typisches Beispiel ist die sogenannte präprothetische Distraktion des Alveolarkammes mit dem Ziel durch Zahnlosigkeit und entsprechende Resorption verlorengegangene Alveolarkammhöhe wieder zu rekonstruieren, um Dentalimplantate einsetzen zu können. Vorrichtungen für solche Anwendungen werden z.B. in den Druckschriften *WO98*/*16163* oder *DE19804316A1* präsentiert.

Diesen Konstruktionen ist der Nachteil gemeinsam, dass sie entweder eine relativ geringe Kontrolle über die Distraktionsrichtung zulassen (z.B. *WO98*/*16163),* konstruktionsbedingt meist unmittelbar an der Distraktionsstelle liegen müssen oder relativ unflexibel sind bezüglich genauer Definition der Distraktionsrichtung. Die genaue Einhaltung einer vorgegebenen Distraktionsrichtung ist aber sehr wichtig, weil nur durch sie effektiv Knochen an dem Ort entsteht, wo er z.B. zur Verankerung eines Dentalimplantates benötigt wird.

Die der Erfindung zugrundeliegende Aufgabe wird durch eine Vorrichtung für kieferorthopädische und/oder kieferchirurgische Zwecke mit den Merkmalen des Anspruchs 1 gelöst. Bevorzugte Ausführungsformen sind in den abhängigen Ansprüchen angegeben.

Diese Vorrichtung, die im Bereiche der Kieferorthopädie und der Kieferchirurgie zum Einsatz kommt, bietet dem Behandler die Möglichkeit, eine stabile, knochenabgestützte Verankerung einzurichten, z.B. um Zähne mit orthodontischen Mitteln im Alveolarkamm zu verschieben und/oder zu kippen, oder um abgetrennte Segmente von Kieferknochen dagegen kontrolliert und zielgerichtet zu distrahieren. Die Vorrichtung beruht auf dem Prinzip, eine von den Zahnwurzeln entfernte Befestigung vorzunehmen und auch in der

Lage zu sein, die Anordnung in einer beliebigen Position entlang des Alveolarkammes bzw. -bogens auszurichten, wobei mehrere, unabhängige Federn an dem Profilelement über entsprechende Halteteile ausgerichtet und fixiert werden können, um z.B. einzelne Zähne gezielt und gleichzeitig zu bewegen. Dank der spezifischen Konstruktion ist es auch möglich, auf die sich während der fortschreitenden Behandlung ändernden Bedürfnisse des Kieferorthopäden mit wenig Aufwand einzugehen. Ein besonderer Vorteil liegt auch darin, dass die Vorrichtung mit dem Profilelement oberhalb der Okklusalebene positioniert werden kann, um während der Behandlung eine Zugkraft auf die entsprechenden Zahn- und Knochenbereiche ausüben zu können.

Die Vorrichtung gemäß der vorliegenden Erfindung kann einerseits abseits vom Ort der Distraktion an chirurgisch optimaler Stelle befestigt werden und erlaubt andererseits durch ihre Konstruktion eine flexible aber stabile Festlegung der Distraktionsrichtung; weiterhin wird auch eine grossflächige, gut kontrollierte Distraktion möglich, wenn z.B. ein Grossteil des Alveolarkammes gleichzeitig vertikal distrahiert werden soll.

Während die Vorrichtung in ihrem Grundaufbau ein Befestigungselement aufweist, an dem das Profilelement gehalten ist, wobei an diesem Profilelement wiederum einzelne Halteteile als Befestigungsstellen aufgesetzt werden können, ist es für umfangreichere kieferchirurgische oder kieferorthopädische Behandlungen von Vorteil, das entsprechend lange Profilelement mit einem zweiten Befestigungselement zu halten derart, dass das Profilelement zwischen zwei dieser Befestigungselemente eingespannt ist. Das eine dieser Befestigungselemente wird im Bereich des ramus ascendens oder des posterioren corpus mandibulae verankert.

Durch ihren relativ einfachen konstruktiven Aufbau und der sich daraus ergebenden geringen Baugröße kann das Befestigungselement entweder am Knochen oder am Zahn verankert werden.

Wie bereits erwähnt, bietet das Profilelement, das entweder an einem Befestigungselement in Form eines auskragenden Arms gehalten ist oder zwischen zwei Befestigungselementen in Form einer Traverse angeordnet ist, die Möglichkeit, an beliebiger Stelle Verankerungspunkte vorzusehen, indem eine entsprechende Anzahl von Halteteilen auf das Profilelement aufgesetzt wird.

Für eine große Variationsmöglichkeit sind bevorzugt die Halteteile an dem Profilelement verschiebbar und mittels Feststellmitteln in der Position fixierbar, wobei die Halteteile für kieferorthopädische Massnahmen und/oder kieferchirurgische Massnahmen ausgelegt sind und die Verankerungspunkte an dem Profilelement bilden.

Das Profilelement kann einen gesamten Zahnbogen umspannend ausgelegt sein, wobei die beiden Enden jeweils ein dem ramus ascendens dexter bzw. dem posterioren corpus mandibulae dexter und dem ramus ascendens sinister oder dem posterioren corpus mandibulae sinister zuordenbares Befestigungselement aufweisen. Auch bei dieser Anordnung kann das jeweilige Befestigungselement am Knochen oder am Zahn verankert werden.

Um beispielsweise die Krümmung des Zahnbogens annähern zu können, kann es von Vorteil sein, dass das Profilelement aus miteinander verschraubten und in der relativen Stellung zueinander einstellbaren Gliederelementen zusammengesetzt wird.

Eine weitere Einstellmöglichkeit, die gerade bei den kieferorthopädischen und kieferchirurgischen Eingriffen wichtig ist, ist dann gegeben, wenn das Profilelement an dem hinteren Befestigungselement mittels Gelenkverbindung angeordnet wird. Eine solche Gelenkverbindung kann ein im Knochen verankerbares Kugelelement, das durch ein dem Profilelement zuordenbares Klemmteil umgriffen wird, aufweisen. Eine andere Möglichkeit zum Aufbau dieser Gelenkverbindung wird durch eine steckbare Verbindung erzielt.

Um diese Gelenkverbindung in ihrer Winkelorientierung beliebig, d.h. in allen Richtungen, einstellen zu können, kann das Befestigungselement die Form einer Schraube mit Kugelkopf annehmen.

Eine bevorzugte Anordnung ist dann gegeben, wenn das hintere Befestigungselement mit mindestens zwei Laschen versehen ist, welche auf der äusseren und inneren Seite des ramus aszendens verlaufen und mindestens auf der einen Seite Löcher für eine oder mehrere Knochenschrauben aufweisen.

In einem weiteren Aufbau kann das Befestigungselement auch eine durch eine Schraube aktivierbare Klammer umfassen, deren Enden um die Hinter- und Vorderkante des ramus aszendens greifen. Dadurch ergibt sich eine Art Klammer; diese Klammer, die U-förmig sein kann, kann dann in der Mitte der Krümmung jeweils einen nach innen gebogenen Dorn aufweisen, der beim Anziehen der Schraube in dem Knochen eingreifend und sich verankernd ausgelegt ist.

Für die Verbindung zwischen Befestigungselement und Profilelement kann eine sogenannte Bride eingesetzt werden.

Das jeweilige Halteteil kann für kieferchirurgische Massnahmen ein Ende in Form einer U-förmigen Klammer, im Querschnitt gesehen, mit zueinander zugewandten freien Enden aufweisen.

Weiterhin kann das jeweilige Halteteil für kieferchirurgische Massnahmen ein Ende in Form eines Stifts aufweisen.

Auch kann das Befestigungselement für kieferchirurgische Zwecke zur lingualen Seite hin ausbuchtend verlaufen.

Wenn das Profilelement mit einem kreisförmigen Querschnitt ausgestattet wird, können die entsprechenden Halteteile, die auf dieses Profilelement aufgesetzt werden, in die erwünschte radiale bzw. winkelmäßige Stellung gedreht werden, um die jeweiligen Anforderungen anzupassen.

Falls eine stabile Lage des Halteteils an dem Profilelement erforderlich ist, sollte das Profilelement einen nicht-kreisförmigen Querschnitt zur Aufnahme eines Moments um seine Längsachse aufweisen. Eine bevorzugte Querschnittsform ist hierbei viereckig oder rechteckig.

Als zusätzliche Haltemaßnahmen der Halteteile an dem Profilelement können Verankerungspunkte vorgesehen werden, die Einraststellen bilden. Solche Verankerungspunkte können durch Nuten oder Vertiefungen gebildet werden.

Auch das Befestigungselement kann einen stabförmigen Abschnitt aufweisen, entlang dem das Profilelement verschiebbar und feststellbar gehalten ist; dadurch ist eine höhenmäßige Orientierung und Ausrichtung möglich.

Für den Fachmann liegen die spezifischen Vorteile, die sich aus den vorstehend geschilderten konstruktiven Maßnahmen ergeben, auf der Hand.

Weitere Vorteile und Merkmale der Erfindung ergeben sich aus der nachfolgenden Beschreibung von Ausführungsbeispielen anhand der Zeichnungen. In der Zeichnung:
- Figur 1a: zeigt eine Draufsicht auf den Unterkiefer mit einem komplett der Bezahnung folgenden Profilelement, wobei die Verbindung zu hinteren, schraubenartigen Knochenbefestigungen über ein Kugelgelenk erfolgt, während die vordere Abstützung über zwei interforaminal gesetzte Knochenplatten mit vertikalen Verbindungszylindern vorgenommen ist;
- Figur 1b: zeigt eine Seitenansicht der Darstellung der Figur 1a aus Richtung des Sichtpfeils 1b in Figur 1.
- Figur 2a: zeigt eine der Figur 1a entsprechende Darstellung, wobei das Halteteil nicht dem gesamten Alveolarkamm folgt; die hintere Befestigung hat die Form einer der Topographie angepassten Knochenplatte mit einem aus der Schleimhaut herausragenden Verbindungsteil bzw. Befestigungselement mit Kugelkopf;
- Figur 2b: zeigt eine weitere, der Figur 1a entsprechende Darstellung, wobei allerdings das Profilelement nur auf den zwei interforaminal befestigten Knochenplatten mit vertikalen Verbindungen abgestützt wird; bei dieser Varianten dient das Profilelement dazu, ein interforaminales Segment des Alveolarkammes hoch zu distrahieren;
- Figur 3a: zeigt eine Variante des hinteren Befestigungselements, bei der zwei Plattenteile aussen am ramus ascendens mit Schrauben befestigt werden, während eine innenliegende Lasche zusätzliche Stabilität verleiht;
- Figur 3b: zeigt eine weitere Darstellung, ähnlich derjenigen der Figur 3, des hinteren Befestigungselements, bei der eine Art Klammer mit einer Schraube am ramus ascendens festgeklemmt wird, ohne Schrauben darin einbohren zu müssen;
- Figur 4a: zeigt eine Ansicht von vorne des Unterkiefers, bei der eine Vorrichtung mit einem komplett umlaufenden Profilelement vorgesehen ist; mit diesem Profilelement wird zuerst ein anteriores Segment des Alveolarkammes vertikal distrahiert und anschliessend können weitere, orthodontische Korrekturen mit Abstützung auf dem Profilelement erfolgen; die Befestigung des anterioren Segmentes erfolgt über ein einem Steigbügel ähnliches Teil;
- Figur 4b: zeigt eine mit der Figur 4a vergleichbare Situation, bei der das zu distrahierende Segment mit Schrauben am vertikalen Distraktor festgehalten ist;
- Figur 4c: zeigt eine zur Figur 4a analoge Situation, bei der ein Segment im lateralen Alveolarkamm vertikal distrahiert wird;
- Figur 4d: zeigt eine Anordnung mit einem Befestigungselement in Form eines U;
- Figur 4e: zeigt eine Anordnung, vergleichbar mit derjenigen der Figur 4d, mit einem direkt an dem Knochen anliegenden Befestigungselement;
- Figur 5: zeigt die Vorrichtung in der Varianten eines kranartigen Distraktors; bei diesem Distraktor wird das Profilelement genau über der Distraktionszone mit genau eingestelltem Distraktiosvektor ausgerichtet;
- Figur 6: zeigt ein Profilelement mit mehreren daran aufgesetzten, unterschiedlich ausgestalteten Halteteilen;
- Figur 7: zeigt die Verbindung zwischen einem Verbinder und einem Profilelement in einer ersten Ausführung;
- Figur 8: zeigt die Verbindung zwischen einem Verbinder und einem Profilelement in einer zweiten Ausführung; und
- Figur 9: zeigt die Verbindung zwischen einem Verbinder und einem Profilelement in einer dritten Ausführung.

In der nachfolgenden Beschreibung wird das Profilelement auch als Reling bezeichnet, insbesondere dann, wenn die Vorrichtung für die segmentale Distraktion und die knochenabgestützte Kieferorthopädie eingesetzt wird, wenn sie auf mindestens zwei Abstützungen aufliegt und die entsprechende, dem Zahnkranz entlanglaufende Form hat. In der nachfolgenden Beschreibung wird die Vorrichtung auch als Krandistraktor bezeichnet, wenn das Profilelement nur eine Knochenabstützung besitzt und gegebenenfalls mit Gelenken versehen ist.

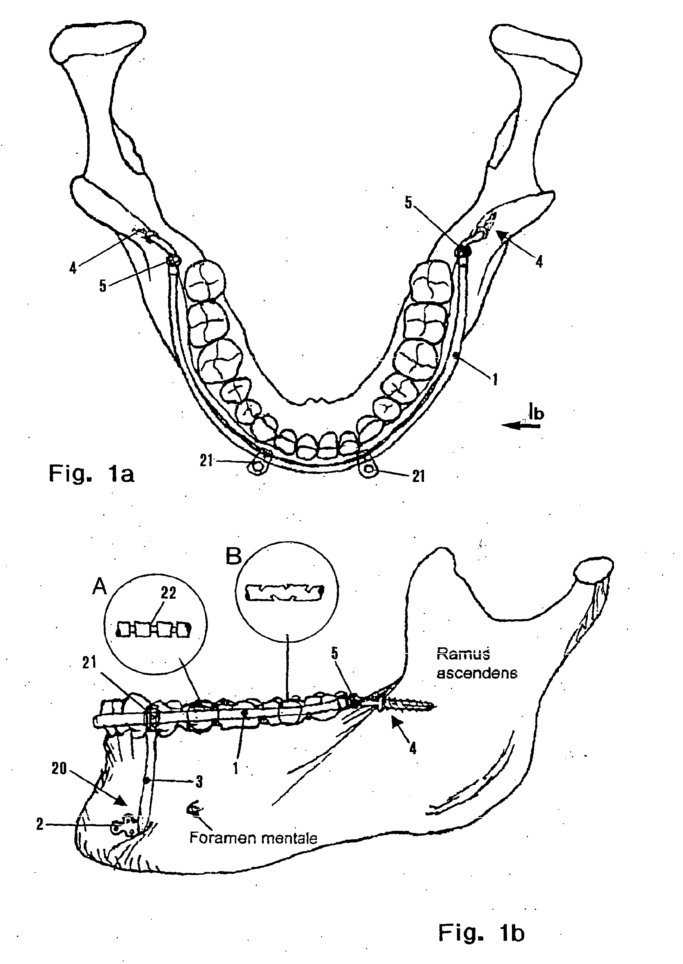

Die Figuren 1a und 1b zeigen die Vorrichtung in der Variante 1 als Reling von oben bzw. von der Seite. Die Reling besteht aus einem in dieser Zeichnung rund um den Zahnbogen geführten Profilelement 1, welches vorne in diesem Fall von zwei interforaminal (d.h. vor dem Nervaustritt oder foramen mentale) platzierten, speziellen Knochenplatten 2 mit vertikalen Verbindern 3 getragen wird. Die jeweilige Knochenplatte 2 und der jeweilige Verbinder 3 bilden ein Befestigungselement 20 für das Profilelement 1. Die vertikalen Verbinder 3 können die Form eines Zylinders annehmen, womit eine Klemmbride 21 am oberen Ende, welche die Reling 1 festhält, in beliebiger Position entlang und um den Zylinder herum platziert werden kann. Der vertikale Verbinder 3 kann aber auch einen rechteckigen oder sonstige Querschnitt annehmen, wodurch es einfacher werden kann, z.B. ein kieferorthopädisches Bracket direkt daran festzulöten oder anzuschweissen.

Das Profilelement 1 kann entweder einen kreisförmigen Querschnitt aufweisen, um wiederum damit eine darauf zu befestigende Klemmverbindung in beliebiger Lage und Position zu befestigen, oder es kann einen nicht-kreisförmigen Querschnitt aufweisen, damit ein Drehmoment um die Längsachse aufgefangen werden kann. In beiden Fällen kann das Profilelement 1 auch mit Vertiefungen 22 versehen werden, damit z.B. kieferorthopädische Drähte oder Brackets direkt daran befestigt werden können. Die Vertiefungen 22 können die Form von umlaufenden Nuten (Detail A), von asymmetrischen Nuteinfräsungen (Detail B) oder von Flächen, worauf ein Bracket angeordnet werden kann, haben. Die Verschiebung und Positionierung einer Klemmverbindung bzw. eines Halteteils auf der Reling 1 wird durch diese Vertiefungen nicht beeinträchtigt.

Falls die Reling bzw. das Profilelement 1 bis zu den hintern Molaren reicht, kann es dort über ein z.B. im ramus ascendens befestigtes Element gehalten werden. In der gezeigten Anordnung handelt es sich z.B. um eine spezielle Knochenschraube 4 mit einem Kugelkopf 5, der wiederum über eine am Ende der Reling 1 angebrachte Pfanne mit schraubenaktivierter Klemmöglichkeit gefasst wird und so eine stabile, bei der Operation leicht einstellbare, Position ermöglicht.

Wie anhand insbesondere der Figur 1 zu erkennen ist, kann über die beiden Befestigungselemente 20 mit den Verbindern 3 und der Klemmbride 21 die Höhe des Profilelements 1 eingestellt werden, derart, dass das Profilelement bzw. die Reling 1 in einer bestimmten Höhe seitlich der Zahnreihe positioniert ist, wie dies die Figur 1b zeigt, oder aber im Bereich der Okklusionsebene oder darüber liegt, um entsprechende kieferchirurgische und kieferorthopädische Maßnahmen vorzunehmen.

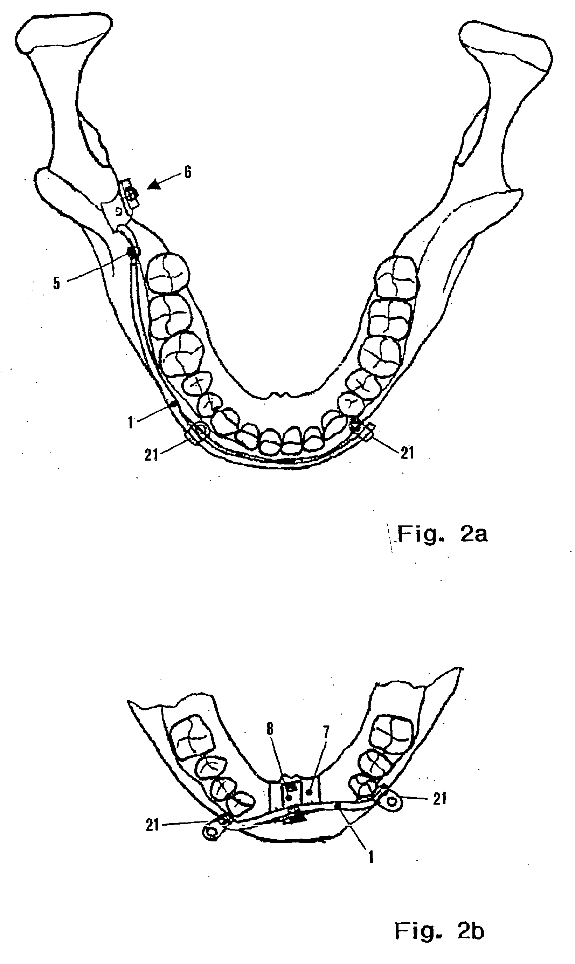

In Figur 2a ist die Reling bzw. das Profilelement 1, im Gegensatz zu der Anordnung der Figur 1a, nicht um den ganzen Zahnkranz gezogen, weil z.B. im linken hinteren Teil des Gebisses keine Behandlung nötig ist. In diesem Beispiel nimmt die hintere Abstützung die Form einer Knochenplatte 6 an, die mit Standardknochenschrauben (nicht näher dargestellt) befestigt wird und vorne als Verbindung zur Reling 1 einen Fortsatz wiederum mit Kugelkopf 5 besitzt. Dieser Kugelkopf 5 wird, wie vorstehend beschrieben, von einer der Reling 1 zugeordneten Pfanne gefasst.

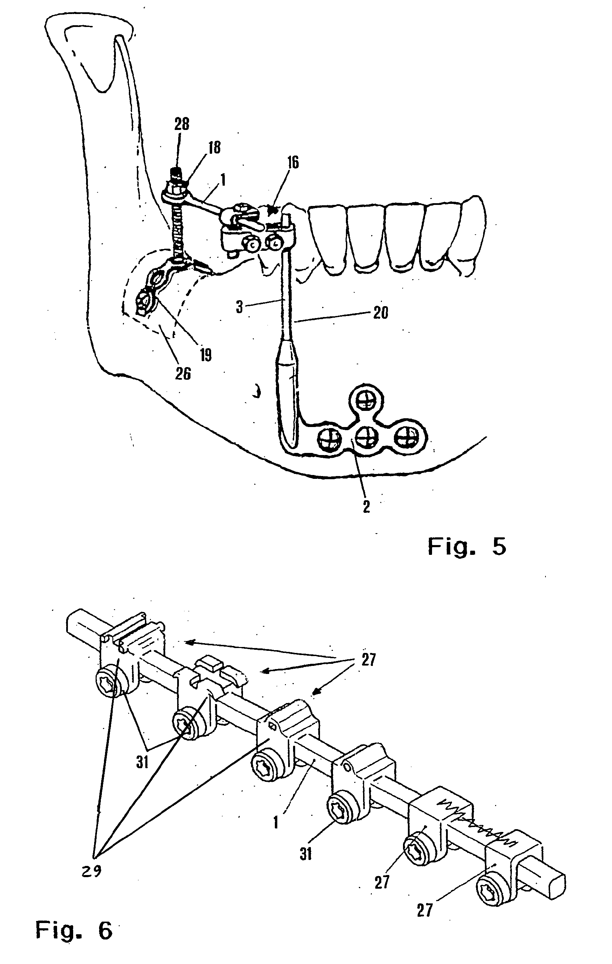

Figur 2b zeigt eine sehr kurze Variante der Reling 1. In diesem Fall dient sie dazu, ein aus dem Alveolarkamm ausgesägtes Knochensegment 7 vertikal distrahieren zu können. Das Knochensegment 7 ist mit üblichen Knochenschrauben (nicht näher dargestellt) an einer Knochenplatte 8 befestigt. Diese Knochenplatte 8 ist über eine verstellbare, vertikale Verbindung mit der Reling 1 verbunden, welche im Sinne einer Distraktion eine graduelle, kontrollierte Bewegung nach oben ermöglicht (siehe hierzu auch die nachfolgend beschriebene Figur 5). Typischerweise kann dies über eine Gewindestange mit einer Stellmutter erfolgen, aber auch über einen flexiblen Drahtzug.

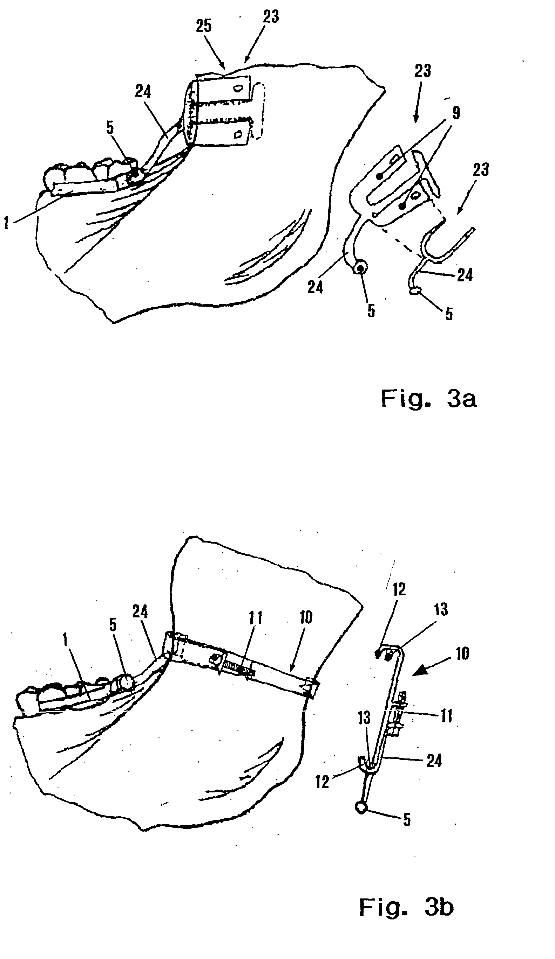

Die Figuren 3a und 3b zeigen weitere spezifische Ausführungen eines hinteren Befestigungselements 23. Wiederum wird die Verbindung zur Reling bzw. zum Profilelement 1 über einen klemmbaren Kugelkopf 5 auf einem kurzen Arm 24 hergestellt, wobei die spezielle Knochenplatte 25 in Figur 3a in der Draufsicht die Form eines "U" annimmt, wie anhand der zwei linken Detaildarstellungen gut zu erkennen ist. Auf der einen Seite sind zwei Laschen 9 mit Löchern für Standardknochenschrauben angebracht, während die andere Seite lediglich Abstützungsfunktion hat, aber durchaus Löcher für eine Befestigung beinhalten könnte.

In der Figur 3b erfolgt die Befestigung über eine zusammenziehbare Klemmschelle 10 mit einem dazugehörigen Arm 24, welcher den Kugelkopf 5 trägt. Die Klemmschelle 10 wird über eine Schraube 11 zusammengezogen. Sie weist an beiden Enden einen Bogen 12 auf mit einem Dorn 13 im Bereich der inneren Krümmung, der sich in den Knochen gräbt. Anstelle der Kugelköpfe 5 an der hintere Befestigung, wie dies in den Figuren 2a, 3a und 3b gezeigt ist, könnte die hintere Befestigungseinheit 10 auch eine Klemmvorrichtung am vorderen Ende des Verbindungsarmes tragen, mit welcher die Reling 1 oder ein ihr zugeordnetes Kupplungselement oder eine Steckvorrichtung festgeklemmt werden könnte.

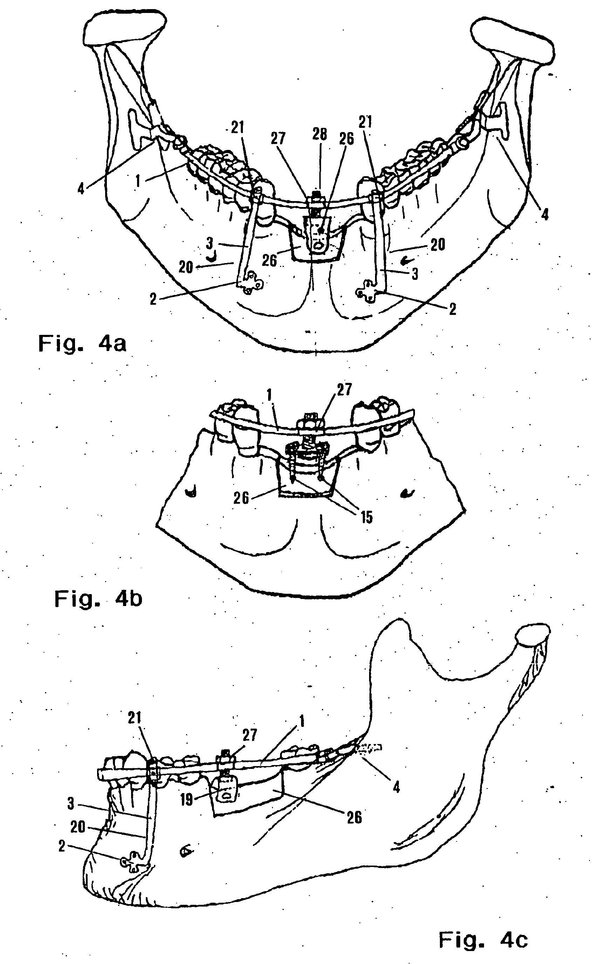

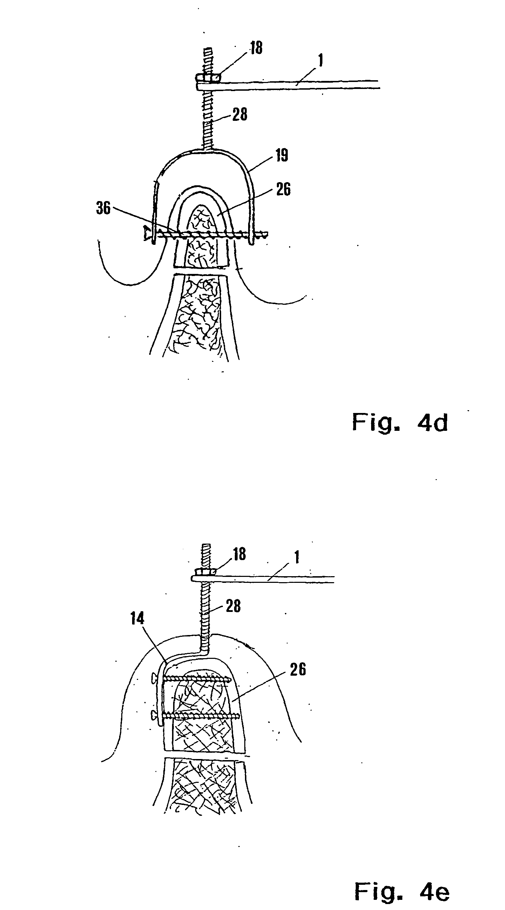

Die Figuren 4a bis 4c zeigen Anwendungen der Reling bzw. des Profilelements 1, die in dem dargestellten Zustand jeweils dazu dienen, ein anteriores Knochensegment 26 vertikal zu distrahieren, z.B. um genügend Knochenhöhe für die Implantation eines Implantates zu gewinnen. Dabei ist an der Reling bzw. dem Profilelement 1 ein Halteteil 27 festgeklemmt, das einen Verstellmechanismus trägt, mit dem das Knochensegment graduell vertikal verschoben werden kann. Ein detailliertes Ausführungsbeispiel mit einem solchen Mechanismus ist in den Figuren 4d, 4e und 5 dargestellt.

Wie in den Figuren 4a, 4b, 4c, und insbesondere auch in den Figuren 4d, 4e und 5, zu sehen ist, erfolgt die Befestigung am Knochensegment 26 über eine mit dem vertikalen Stellmechanismus verbundene lineare Knochenplatte 14, die, je nach Einsatzbereich, entsprechend der Form, beispielsweise des Alveolarkammes, in U- bzw. Steigbügelform gebogen ist, wie dies insbsondere auc die Figur 4d verdeutlicht. Dieser Verstellmechanismus weist eine Gewindestange 28 auf, die an dem Profilelement mit einer Mutter 18 gehalten ist. Durch Anziehen der Mutter 18 wird die Gewindestange nach oben gezogen, um die erwünschte Distraktion vorzunehmen. In der Ausführungsform der Figur 4d ist zu erkennen, dass das Knochensegment 26 mittels einer durch die beiden Enden der Schenkel des Steigbügels hindurchgeführten und sich im Knochen verankerten Schraube 36 gehalten ist. Anhand der Figur 4d ist auch zu erkennen, dass die Halterung in Form des Steigbügels 19 außerhalb der Gingiva bzw. Mukosa liegt.

Im Gegensatz zu de Ausführungsform der Figur 4d zeigt die Figur 4e eine Ausführungsform, bei der mit der Gewindestange 28 eine Knochenplatte 14 verbunden ist, die direkt auf dem Knochensegment 26 aufliegt, d.h. einer Form angepasst ist. Diese Knochenplatte 14 ist mit zwei Schrauben im Knochen verschraubt. Somit liegt diese Knochenplatte unterhalb der Gingiva (ist subgingival) oder unterhalb der Mukosa (ist submukös).

In Figur 4b erfolgt die Befestigung nicht über die steigbügelförmige Platte, sondern über im Stellmechanismus direkt integrierte Knochenschrauben 15, die sich in das Knochensegment 26 einbohren.

Figur 5 zeigt neben dem bereits angesprochenen Mechanismus mit Gewindestange 28 eine Anwendung, in der die Vorrichtung die Form eines Krans annimmt und auf eine hintere Abstützung verzichtet. Das Profilelement 1 ist durch miteinander verschraubte und in der relativen Stellung zueinander einstellbare Gliederelemente, mit dem Bezugszeichen 16 bezeichnet, erweitert. Durch diese Gliederelemente, auch als Bridenkette 16 bezeichnet, ist es möglich, die vertikale Verstelleinheit genau über das zu distrahierende Knochensegment 26 zu bringen und es in einem genau definierbaren Vektor anzuheben. Im vorliegenden Beispiel umfasst die vertikale Verstelleinheit wieder den bereits angesprochenen Mechanismus mit Gewindestange 28 und zughöriger Stellmutter 18. Die Befestigung des Knochensegmentes 26 erfolgt über eine der Kammform angebogene Platte 19 in Bügelform. Die Platte liegt extramukosal, d.h. außerhalb der Gingiva.

Durch ihre Anpassbarkeit ermöglicht es diese Konstruktion, Knochensegmente präzise und genau geführt vertikal zu distrahieren und gleichzeitig die Befestigung interforaminal, d.h. vor dem Nervaustritt vorzunehmen, wodurch eine Gefährdung des nervus mentalis durch die Knochenschrauben der Ankerplatte vermieden werden kann.

Zusätzlich zu den in den Figuren 1 bis 5 gezeigten Klemmverbindungen können zusätzliche Gelenke eingebaut werden, die den vertikalen Distraktor aus der Vertikalen abwinkeln können, so dass ein leicht schiefer Distraktionsvektor möglich wird.

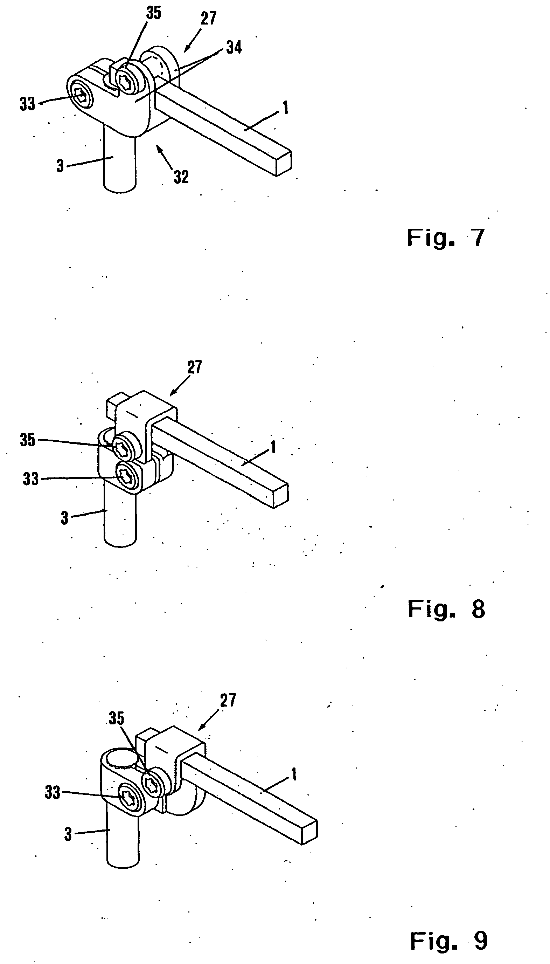

Klemmverbindungen sind in den Figuren 7, 8 und 9 gezeigt, während Figur 6 ein Profilelement 1 zeigt mit einzelnen Halteteilen 27. Die Halteteile 27 umfassen ein im Wesentlichen U-förmiges Grundelement 29, durch dessen beide freien Schenkel 30 eine Klemmschraube 31 hindurchgeführt ist. Mit dieser Klemmschraube 31 können die beiden freien Schenkel 30 gegeneinander verspannt werden, um das Halteteil 27 an dem im Querschnitt quadratisch ausgebildeten Profilelement 1 zu verklemmen. Zu jedem Zeitpunkt können diese Halteteile 27 durch Lösen der Klemmschraube 31 verschoben und umorientiert werden. Die einzelnen Halteteile 27 besitzen unterschiedliche Formen, um eine Verankerung von Verankerungsteilen vorzunehmen; hierzu sind Durchgangsbohrungen, Schlitze und Nuten gezeigt, um einige Beispiele anzuführen.

In Figur 6 sind die beiden am weitesten rechts auf das Profilelement aufgesetzten Briden über eine Feder miteinander verbunden, um eine Möglichkeit zu zeigen, bei der die eine Klemmbride, in dem vorliegenden Fall die rechte der beiden mit Federn verbundenen Briden, an dem Profilelement 1 festgeklemmt ist, während die links liegende, mit dem Federelement verbundene Bride gleitend angeordnet ist; mit einer solchen Anordnung ist die frei gleitbare, links liegende Bride mit der Federkraft beaufschlagt, um einen entsprechenden Zug, oder gegebenenfalls auch eine Druckwirkung, auf diese Bride für den bestimmten Behandlungsfall aufzubringen.

Figur 7 zeigt einen Verbinder 3 und ein Profilelement 1 mit rechteckigem Querschnitt, wobei diese Teile durch eine L-förmige Bride 32 im rechten Winkel zueinander gehalten sind. Der eine Schenkel der Bride 32 ist geschlitzt, um ihn über eine Stellschraube 33 an dem Verbinder 3 festzuklemmen, während der andere Schenkel zwei Laschen 34 aufweist, zwischen denen das Profilelement 1 eingelegt ist, und die mittels einer Stellschraube 35 gegeneinander verklemmbar sind. Das Profilelement 1 kann zwischen den Laschen 34 nach Lösen der Stellschraube zu einer erwünschten Lage und Position verschoben werden.

Figur 8 zeigt eine Ausführungsform des Halteteils 27, das wiederum eine L-förmige Grundform in der Seitenansicht zeigt, um mit dem einen Schenkel den Verbinder 3 aufzunehmen und mit dem anderen Schenkel das Profilelement 1, wobei diese beiden Teile jeweils mit den Stellschrauben 33 und 35 fixiert werden können. Die Stellschrauben sind in dieser Ausführungsform übereinander angeordnet, wodurch die Baugröße dieses Halteteils 27 verringert werden kann.

Figur 9 zeigt schließlich ein Halteteil 27, mit einer in der Seitenansicht L-förmigen Grundform, bei der allerdings die beiden Schenkel über die eine Stellschraube 33, die das Halteteil 27 auch an dem Verbinder 3 festklemmt, verschwenkt werden können, um dadurch die Winkelstellung der beiden Schenkel des Halteteils 27 und damit die Winkelstellung des Verbinders 3 und des Profilelements 1 zueinander einzustellen. Es ist auch ersichtlich, dass das Halteteil 27 durch Lösen der entsprechenden Stellschraube 33 um die Achse des Verbinders 3 geschwenkt werden kann, so dass auch in dieser radialen Richtung um den Verbinder 3 herum eine Ausrichtung des Profilelements ermöglicht wird.

## Patentansprüche

1. Vorrichtung für kieferorthopädische und/oder kieferchirurgische Zwecke im Bereich des Unterkiefers mit einem Profilelement (1), das mit zumindest einem vorderen, in der Regel dem interforaminalen Bereich eines Unterkiefers oder der anterioren mandibula zuordenbaren, im Knochen verankerbaren Befestigungselement (20) gehalten ist, wobei das Befestigungselement (20) aus einer am interforaminalen Bereich eines Unterkiefers oder einer der anterioren mandibula zuordenbaren, im Knochen verankerbaren, eine stabile Knochenabstützung bildenden Knochenplatte (2) und aus einem vertikalen Verbinder (3) aufgebaut ist,
**dadurch gekennzeichnet,**
**dass** das Profilelement (1) eine in zumindest Teilbereichen der vestibulären Seite eines Zahnbogens angenäherte Krümmung aufweist, und wobei das Profilelement (1) mindestens einen Verankerungspunkt, an dem ein erstes Halteteil (16; 21; 29) angreift, aufweist, und dass an dem vertikalen Verbinder (3) im Bereich des oberen, der Knochenplatte (2) gegenüberliegenden Endes als erstes Halteteil (16; 21; 29) eine Klemmbride (21) angeordnet ist, an der das Profilelement (1), einen horizontalen, auskragenden, langgestreckten, formstabilen, selbsttragenden Arm zu dem vertikalen Verbinder (3) bildend, in der Höhe an dem vertikalen Verbinder (3) einstellbar, gehalten ist, mit einer Länge des Verbinders (3) derart, dass über ein an dem Profilelement (1) gehaltenes zweites Halteteil (27; 28) eine segmentale Distraktion in vertikaler Richtung von Unterkieferknochen gegenüber diesem zweiten Halteteil ausführbar ist.

2. Vorrichtung nach Anspruch 1, **dadurch gekennzeichnet, dass** ein weiteres, hinteres, im Bereich des ramus ascendens oder des posterioren corpus mandibulae verankerbares Befestigungselement (4) vorgesehen ist.

3. Vorrichtung nach Anspruch 2, **dadurch gekennzeichnet, dass** das hintere Befestigungselement (4) am Knochen verankert ist.

4. Vorrichtung nach Anspruch 2, **dadurch gekennzeichnet, dass** das hintere Befestigungselement am Zahn verankert ist.

5. Vorrichtung nach Anspruch 1, **dadurch gekennzeichnet, dass** mehrere einzelne Verankerungspunkte an dem Profilelement durch mehrere daran anordenbare Halteteile (21; 29) gebildet sind.

6. Vorrichtung nach Anspruch 5, **dadurch gekennzeichnet, dass** die Halteteile an dem Profilelement (1) verschiebbar sind.

7. Vorrichtung nach Anspruch 6, **dadurch gekennzeichnet, dass** das jeweilige Halteteil (21; 29) mittels Feststellmitteln (31) in der Position fixierbar ist, wobei die Halteteile (21; 29) für kieferorthopädische Massnahmen und/oder kieferchirurgische Massnahmen ausgelegt sind und die Verankerungspunkte an dem Profilelement (1) bilden.

8. Vorrichtung nach Anspruch 1, **dadurch gekennzeichnet, dass** das Profilelement (1) einen gesamten Zahnbogen umspannend ausgelegt ist und dass die beiden Enden jeweils ein dem ramus ascendens dexter bzw. dem posterioren corpus mandibulae dexter und dem ramus ascendens sinister oder dem posterioren corpus mandibulae sinister zuordenbares Befestigungselement aufweisen.

9. Vorrichtung nach Anspruch 8, **dadurch gekennzeichnet, dass** das jeweilige Befestigungselement (20) am Knochen verankert ist.

10. Vorrichtung nach Anspruch 1, **dadurch gekennzeichnet, dass** das Profilelement (1) aus miteinander verschraubten und in der relativen Stellung zueinander einstellbaren Gliederelementen (16) zusammengesetzt ist.

11. Vorrichtung nach Anspruch 2, **dadurch gekennzeichnet, dass** das Profilelement (1) an dem hinteren Befestigungselement (4) mittels Gelenkverbindung (5) angeordnet ist.

12. Vorrichtung nach Anspruch 11, **dadurch gekennzeichnet, dass** die Gelenkverbindung (5) ein im Knochen verankerbares Kugelelement, das durch ein dem Profilelement (1) zuordenbares Klemmteil umgriffen wird, aufweist.

13. Vorrichtung nach Anspruch 11, **dadurch gekennzeichnet, dass** die Gelenkverbindung durch eine steckbare Verbindung gebildet ist.

14. Vorrichtung nach Anspruch 2 und 11, **dadurch gekennzeichnet, dass** das hintere Befestigungselement die Form einer Schraube (4) mit Kugelkopf (5) annimmt.

15. Vorrichtung nach Anspruch 1 **dadurch gekennzeichnet, dass** das hintere Befestigungselement mit mindestens zwei Laschen (9) versehen ist, welche auf der äusseren und inneren Seite des ramus ascendens verlaufen und mindestens auf der einen Seite Löcher für eine oder mehrere Knochenschrauben aufweisen.

16. Vorrichtung nach Anspruch 2, **dadurch gekennzeichnet, dass** das hintere Befestigungselement eine durch eine Schraube aktivierbare Klammer umfasst, deren Enden um die Hinter- und Vorderkante des ramus aszendens greifen.

17. Vorrichtung nach Anspruch 16 **dadurch gekennzeichnet, dass** die hintere und vordere Klammer in der Mitte der Krümmung jeweils einen nach innen gebogenen Dorn (13) aufweisen, der beim Anziehen der Schraube in dem Knochen eingreifend und sich verankernd ausgelegt ist.

18. Vorrichtung nach Anspruch 1, **dadurch gekennzeichnet, dass** das vordere Befestigungselement mittels einer Bride mit dem Profilelement (1) verbunden wird.

19. Vorrichtung nach Anspruch 5, **dadurch gekennzeichnet, dass** das jeweilige Halteteil für kieferchirurgische Massnahmen ein Ende In Form einer U-förmigen Klammer, im Querschnitt gesehen, mit zueinander zugewandten freien Enden aufweist.

20. Vorrichtung nach Anspruch 19, **dadurch gekennzeichnet, dass** das jeweilige Halteteil für kieferchirurgische Massnahmen ein Ende in Form eines Stifts aufweist.

21. Vorrichtung nach Anspruch 1, **dadurch gekennzeichnet, dass** das Befestigungselement für kieferchirurgische Zwecke zur lingualen Seite hin ausbuchtend verläuft.

22. Vorrichtung nach Anspruch 1, **dadurch gekennzeichnet, dass** das Profilelement (1) einen kreisförmigen Querschnitt aufweist.

23. Vorrichtung nach Anspruch 1, **dadurch gekennzeichnet, dass** das Profilelement einen nicht-kreisförmigen Querschnitt aufweist zur Aufnahme eines Moments um seine Längsachse.

24. Vorrichtung nach Anspruch 1, **dadurch gekennzeichnet, dass** das Profilelement (1) Verankerungspunkte aufweist, die eine Einraststelle (22) bilden.

25. Vorrichtung nach Anspruch 24, **dadurch gekennzeichnet, dass** die Verankerungspunkte durch Nuten gebildet sind.

26. Vorrichtung nach Anspruch 25, **dadurch gekennzeichnet, dass** die Verankerungspunkte durch Vertiefungen (22) gebildet sind.

## Claims

1. A device for orthodontic and/or orosurgical purposes in the region of the submaximalla, comprising a profiled element (1) held by at least one front fixing element (20) that can be anchored in the bone and is allocatable as a rule to the interforaminal region of the submaximalla or to the anterior mandible, said fixing element (20) being composed of a bone plate (2) that can be anchored in the bone and forms a stable bone support and is allocatable to the interforaminal region of the submaximalla or to the anterior mandible, and of a vertical connector (3),
**characterized in**
**that** said profiled element (1) has at least some sections that are curved and approximate to the vestibular side of a dental arch, and said profiled element (1) forming at least one anchorage point, upon which a first retaining member (16; 21; 29) is acting, and that said vertical connector (3) in the region of the upper end opposite to said bone plate (2) has arranged thereon as a first retaining member (16; 21; 29) a clamping strap (21) on which said profiled element (1), forming a horizontal, projecting, elongated, dimensionally stable, self-supporting arm with respect to said vertical connector (3), is held to be adjustable in height on said vertical connector (3), with a length of said connector (3) in such a way that via a second retaining member (27; 28) held on said profiled element (1) a segmental distraction in vertical direction of submaximalla relative to said second retaining member can be carried out.

2. The device according to claim 1, **characterized in that** there is provided a further rear fixing element (4) that can be anchored in the region of the ramus ascendens or of the posterior corpus mandibulae.

3. The device according to claim 2, **characterized in that** said rear fixing element (4) is anchored to the bone.

4. The device according to claim 2, **characterized in that** said rear fixing element is anchored to the tooth.

5. The device according to claim 1, **characterized in that** a plurality of individual anchorage points are formed on said profiled element by a plurality of retaining members (21; 29) that can be arranged thereon.

6. The device according to claim 5, **characterized in that** said retaining members are displaceable on said profiled element (1).

7. The device according to claim 6, **characterized in that** said respective retaining member (21; 29) can be fixed by locking means (31) in its position, said retaining members (21; 29) being designed for orthodontic and/or orosurgical measures and forming said anchorage points on said profiled element (1).

8. The device according to claim 1, **characterized in that** said profiled element (1) is designed to span a whole dental arch, and that each of the two ends comprises a fixing element that can be allocated to the ramus ascendens dexter or to the posterior corpus mandibulae dexter and to the ramus ascendens sinister or to the posterior corpus mandibulae sinister.

9. The device according to claim 8, **characterized in that** the respective fixing element (20) is anchored to the bone.

10. The device according to claim 1, **characterized in that** said profiled element (1) is composed of link elements (16) that can be screwed to one another and are adjustable in their relative position to one another.

11. The device according to claim 2, **characterized in that** said profiled element (1) is arranged on said rear fixing element (4) by means of an articulation (5).

12. The device according to claim 11, **characterized in that** said articulation (5) comprises a ball element that can be anchored in the bone and is gripped around by a clamp member which can be allocated to said profiled element (1).

13. The device according to claim 11, **characterized in that** said articulation is formed by a plug-type connection.

14. The device according to claim 2 and 11, **characterized in that** said rear fixing element assumes the shape of a screw (4) with a ball head (5).

15. The device according to claim 1, **characterized in that** said rear fixing element is provided with at least two brackets (9) that extend on the exterior and interior side of the ramus ascendens and comprise holes for one or more bone screws at least at the one side.

16. The device according to claim 2, **characterized in that** said rear fixing element comprises a clip that can be activated by a screw and has ends gripping around the rear and front edge of the ramus ascendens.

17. The device according to claim 16, **characterized in that** said rear and front clips are each provided in the middle of the curvature with an inwardly bent mandrel (13) which is configured to engage into and anchor itself in the bone upon tightening of said screw.

18. The device according to claim 1, **characterized in that** said front fixing element is connected by means of a strap to said profiled element (1).

19. The device according to claim 5, **characterized in that** for orosurgical measures each of said retaining members comprises an end in the form of a clip, which is U-shaped when viewed in cross-section, with free ends facing one another.

20. The device according to claim 19, **characterized in that** for orosurgical measures each of said retaining members has an end in the form of a pin.

21. The device according to claim 1, **characterized in that** for orosurgical purposes said fixing element extends to the lingual side in a bulging manner.

22. The device according to claim 1, **characterized in that** said profiled element (1) has a circular cross-section.

23. The device according to claim 1, **characterized in that** said profiled element has a non-circular cross-section for receiving a moment around its longitudinal axis.

24. The device according to claim 1, **characterized in that** said profiled element (1) has anchorage points forming a locking place (22).

25. The device according to claim 24, **characterized in that** said anchorage points are formed by grooves.

26. The device according to claim 25, **characterized in that** said anchorage points are formed by recesses (22).

## Revendications

1. Dispositif d'orthodontie et de chirurgie maxillo-faciale dans la zone du maxillaire inférieur avec un élément profilé (1) qui est maintenu par au moins un élément avant de fixation (20) pouvant être associé en général à la zone interforaminale du maxillaire inférieur ou de la mandibule antérieure et pouvant être ancré dans l'os, l'élément de fixation (20) étant constitué d'une plaque vissée (2) pour os formant un appui stable pour l'os, qui peut être ancrée dans l'os ou être associée dans la zone interforaminale du maxillaire inférieur ou à l'une des mandibules antérieures, et d'un connecteur vertical (3),
**caractérisé en ce que** l'élément profilé (1) présente une courbure s'approchant de la face vestibulaire de l'arcade dentaire dans au moins des zones partielles et l'élément profilé (1) présentant au moins un point d'ancrage sur lequel s'engage une première pièce de retenue (16; 21; 29) et **en ce que** sur le connecteur vertical (3) dans la zone de l'extrémité supérieure opposée à la plaque vissée (2) pour os, il est disposé en tant que première pièce de retenue (16; 21; 29) une bride de serrage (21) sur laquelle l'élément profilé (1) est maintenu de manière réglable en hauteur sur le connecteur vertical (3), élément qui forme un bras horizontal, faisant saillie, allongé, de forme stable, autoportant par rapport au connecteur vertical (3), avec une longueur du connecteur (3) de sorte que par une seconde pièce de retenue (27; 28) maintenue sur l'élément profilé (1), une distraction segmentaire dans le sens vertical est exécutable par l'os de la mandibule inférieure par rapport à cette seconde pièce de retenue.

2. Dispositif selon la revendication 1, **caractérisé en ce qu'**il est prévu un autre élément de fixation (4) arrière ancrable dans la zone de la branche ascendante (ramus ascendens) ou du corps postérieur de la mandibule (posterior corpus mandibulae).

3. Dispositif selon la revendication 2, **caractérisé en ce que** l'élément de fixation arrière (4) est ancré à l'os.

4. Dispositif selon la revendication 2, **caractérisé en ce que** l'élément de fixation arrière est ancré à la dent.

5. Dispositif selon la revendication 1, **caractérisé en ce que** plusieurs points d'ancrage séparés sont formés sur l'élément profilé par plusieurs pièces de retenue (21; 29) pouvant y être disposées.

6. Dispositif selon la revendication 5, **caractérisé en ce que** les pièces de retenue sont coulissantes sur l'élément profilé (1).

7. Dispositif selon la revendication 6, **caractérisé en ce que** la pièce de retenue respective (21; 29) est fixable en position à l'aide de moyens de fixation (31), les pièces de retenue (21; 29) étant conçues pour des interventions en orthopédie et/ou en chirurgie maxillo-faciale et formant les points d'ancrage sur l'élément profilé (1).

8. Dispositif selon la revendication 1, **caractérisé en ce que** l'élément profilé (1) est réalisé pour encercler une arcade dentaire entière et **en ce que** les deux extrémités présentent un élément de fixation associable à la branche ascendante droite (ramus ascendens dexter) respectivement au corps postérieur de la mandibule droite (posterior corpus mandibulae dexter) et à la branche ascendante gauche (ramus ascendens sinister) ou au corps postérieur de la mandibule gauche(posterior corpus mandibulae sinister).

9. Dispositif selon la revendication 8, **caractérisé en ce que** l'élément de fixation respectif (20) est ancré dans l'os.

10. Dispositif selon la revendication 1, **caractérisé en ce que** l'élément profilé (1) se compose d'éléments d'organe (16) vissés les uns aux autres et réglables les uns par rapport aux autres dans la position relative.

11. Dispositif selon la revendication 2, **caractérisé en ce que** l'élément profilé est disposé sur l'élément arrière de fixation (4) au moyen d'une connexion articulée (5).

12. Dispositif selon la revendication 11, **caractérisé en ce que** la liaison articulée (5) présente un élément sphérique ancrable dans l'os qui est enserré par une pièce de serrage associable à l'élément profilé (1).

13. Dispositif selon la revendication 11, **caractérisé en ce que** la liaison articulée est constituée par une connexion emboîtable.

14. Dispositif selon la revendication 2 et 11, **caractérisé en ce que** l'élément de fixation arrière prend la forme d'une vis (4) à tête sphérique (5).

15. Dispositif selon la revendication 1, **caractérisé en ce que** l'élément de fixation arrière est muni au moins de deux languettes (9) qui s'étendent sur les faces internes et externes de la branche ascendante (ramus ascendens) et présentent, sur au moins l'une des faces, des trous pour une ou plusieurs vis pour os.

16. Dispositif selon la revendication 2, **caractérisé en ce que** l'élément de fixation comprend un crochet actionnable par une vis, dont les extrémités entourent les bords arrière et avant de la branche ascendante (ramus ascendens).

17. Dispositif selon la revendication 16, **caractérisé en ce que** le crochet arrière et avant présente, au milieu de la courbure, respectivement une cheville (13) arquée vers l'intérieur qui est prévue pour pénétrer et s'ancrer dans l'os lors du serrage de la vis.

18. Dispositif selon la revendication 1, **caractérisé en ce que** l'élément avant de fixation est relié à l'élément profilé (1) au moyen d'une bride.

19. Dispositif selon la revendication 5, **caractérisé en ce que** la pièce de retenue respective pour des interventions en chirurgie maxillo-faciale présente une extrémité en forme de crochet en U vue en coupe transversale avec des extrémités libres tournées les unes vers les autres.

20. Dispositif selon la revendication 19, **caractérisé en ce que** la pièce de retenue respective pour des interventions en chirurgie maxillo-faciale présente une extrémité en forme de tenon.

21. Dispositif selon la revendication 1, **caractérisé en ce que** l'élément de fixation pour des mesures en chirurgie maxillo-faciale s'étend en formant une excavation en direction de la face linguale.

22. Dispositif selon la revendication 1, **caractérisé en ce que** l'élément profilé (1) présente une section transversale circulaire.

23. Dispositif selon la revendication 1, **caractérisé en ce que** l'élément profilé présente une section transversale non circulaire pour absorber un moment autour de son axe longitudinal.

24. Dispositif selon la revendication 1, **caractérisé en ce que** l'élément profilé (1) présente des points d'ancrage qui forment un emplacement d'encliquetage (22).

25. Dispositif selon la revendication 24, **caractérisé en ce que** les points d'ancrage sont formés par des rainures.

26. Dispositif selon la revendication 25, **caractérisé en ce que** les points d'ancrage sont formés par des cavités (22).
